# EUROPEAN PATENT SPECIFICATION

(11) **EP 4 000 421 B1**
(45) Date of publication and mention of the grant of the patent: **01.01.2025**
(21) Application number: 21208822.3
(22) Date of filing: 17.11.2021
(51) Int. Cl.: A23L 33/105, A61K 36/05, A61P 1/00, A61P 1/04

(54) **COMPOSITION FOR USE IN AMELIORATING INFLAMMATORY BOWEL DISEASE CONTAINING TISOCHRYSIS LUTEA**
ZUSAMMENSETZUNG ZUR VERWENDUNG IN DER VERBESSERUNG VON ENTZÜNDLICHEN DARMERKRANKUNGEN MIT TISOCHRYSIS LUTEA
COMPOSITION POUR L'UTILISATION DANS L'AMÉLIORATION DE MALADIE INTESTINALE INFLAMMATOIRE CONTENANT DU TISOCHRYSIS LUTEA

(30) Priority: 18.11.2020 KR 20200154187
(43) Date of publication of application: 25.05.2022
(73) Proprietor: Korea Institute of Science and Technology, Seoul 02792 (KR)
(72) Inventor: PAN, Cheol Ho, 25451 Gangwon-do (KR); KIM, Jin Chul, 25451 Gangwon-do (KR); LEE, Eun Ha, 25451 Gangwon-do (KR); HONG, Sung Chul, 25451 Gangwon-do (KR); KIM, Ju Yong, 25451 Gangwon-do (KR); LEE, Eun Young, 25451 Gangwon-do (KR)
(74) Representative: advotec.

(56) References cited:
- US-A1- 2020 268 816
- LUÍSA CUSTÓDIO ET AL: "Fatty acid composition and biological activities of Isochrysis galbana T-ISO, Tetraselmis sp. and Scenedesmus sp.: possible application in the pharmaceutical and functional food industries", JOURNAL OF APPLIED PHYCOLOGY., vol. 26, no. 1, 30 August 2013 (2013-08-30), NL, pages 151 - 161, XP055220822, ISSN: 0921-8971, DOI: 10.1007/s10811-013-0098-0
- TALERO ELENA ET AL: "Bioactive Compounds Isolated from Microalgae in Chronic Inflammation and Cancer", MARINE DRUGS, vol. 13, no. 10, 1 October 2015 (2015-10-01), Basel, CH, pages 6152 - 6209, XP055910364, ISSN: 1660-3397, DOI: 10.3390/md13106152
- NUÑO K ET AL: "Effects of the marine microalgae Isochrysis galbana and Nannochloropsis oculata in diabetic rats", JOURNAL OF FUNCTIONAL FOODS, ELSEVIER BV, NL; INTERNATIONAL FOOD INFORMATION SERVICE (IFIS), FRANKFURT-MAIN, DE, vol. 5, no. 1, 1 January 2013 (2013-01-01), pages 106 - 115, XP002739591, ISSN: 1756-4646, [retrieved on 20121011], DOI: 0.1016/J.JFF.2012.08.011
- BIGAGLI ELISABETTA ET AL: "A Comparative In Vitro Evaluation of the Anti-Inflammatory Effects of a Tisochrysis lutea Extract and Fucoxanthin", MARINE DRUGS, vol. 19, no. 6, 1 January 2021 (2021-01-01), Basel, CH, pages 334, XP055909994, ISSN: 1660-3397, DOI: 10.3390/md19060334

## Description

### BACKGROUND

### (a) Technical Field

The present invention relates to a composition for use in ameliorating, preventing or treating an inflammatory bowel disease containing *Tisochrysis lutea* as an active ingredient.

### (b) Background Art

Inflammatory bowel disease (IBD) is an incurable disease in which chronic inflammation or ulceration occurring in the mucous membranes of the large and small intestines causes continuous diarrhea and bloody stool over the long term, and subsequent relapses occur.

Inflammatory bowel disease is more common among Westerners, but has rapidly become increasingly prevalent in Korea since the 1980s. The age of onset of inflammatory bowel disease is 15-35 years old, inflammatory bowel disease is reported to be prevalent among all age groups, 15% of patients are over 60 years old, and about 15% of patients suffering from inflammatory bowel disease have a family history thereof pertaining to an immediate family member.

Inflammatory bowel disease may be caused by environmental, genetic and immune factors, and the exact etiology thereof remains unclear.

Although the cause of inflammatory bowel disease is still unclear, the activation of inflammatory mediators and immune cells is presumed to be a critical etiology due to autoimmune diseases as well as environmental or genetic factors.

Inflammatory bowel disease is classified into two diseases, namely, ulcerative colitis and Crohn's disease, which are clinically similar but differ from each other with regard to histological findings and endoscopic and immunological aspects, and critical etiologies of these inflammatory bowel diseases are known to be activation of inflammatory mediators and immune cells.

Persistent or inappropriate activation of the intestinal immune system greatly affects the pathophysiology of chronic mucositis inflammation, and eventually leads to mucosal destruction and ulceration, particularly due to infiltration of neutrophils, macrophages, lymphocytes and mast cells.

Inflammatory-promoting cytokines such as TNF-α (tumor necrosis factor-α), interleukin-6 (IL-6), and interleukin-8 (IL-8) play a major role in inflammatory reactions that generally occur in the course of pathogenesis of inflammatory bowel disease. In particular, TNF-α is highly expressed in the colonic lumen and colonic epithelial cells of ulcerative colitis patients, and recent studies have reported that TNF-α is known to act as an important etiology of ulcerative colitis. The anti-TNF-α antibody infliximab is known to be effective not only for the treatment of boils, but also for the treatment of Crohn's disease, which has previously been untreatable. However, such treatment is expensive and causes side effects such as fluid reactions or infectious complications in some patients.

Currently used therapeutic agents for inflammatory bowel disease include 5-aminosalicylic acid (5-ASA) drugs such as sulfasalazine and mesalazine, which block the production of prostaglandins, steroidal immunosuppressants, and immunosuppressants such as azathioprine, 6-mercaptopurine, and cyclosporin in cases not responsive to steroid treatment, and the like.

However, most of these drugs are composed of anti-inflammatory agents or immunosuppressants. Therefore, a prescription similar to that in the case of autoimmune disease is made, and the main treatment is to block inflammation using TNF-alpha, aminosalicylic acid, steroids and the like. However, both inflammation inhibition and mucosal regeneration are required for effective treatment of inflammatory bowel disease.

In particular, when neither inflammation inhibition nor mucosal regeneration occurs in the treatment of inflammatory bowel disease, there is a high probability that additional intestinal disease will occur. Inflammation inhibition can prevent further infection and spread of infection, and at the same time, mucosal regeneration can restore an immune system that has been disrupted by inflammatory bowel disease and restore the changed intestinal flora to a normal state.

The publication of LUISA CUSTODIO et al.: "Fatty acid composition and biological activities of Isochrysis galbana T-ISO, Tetraselmis sp. and Scenedesmus sp.: possible application in the pharmaceutical and functional food industries", in the JOURNAL OF APPLIED PHYCOLOGY, vol. 26, no. 1, 30 August 2013, pages 151-161, evaluated the potential of three different species of microalgae, namely Isochrysis galbana T-ISO (=Tisochrysis lutea), Tetraselmis sp. and Scenedesmus sp. of their use as functional food and/or as source of biomolecules. It is shown that the acetone extract of I. galbana T-ISO significantly reduced the viability of human hepatic carcinoma HepG2 cells. It is concluded that the microalgae tested could be used as functional food and/or source of nutraceuticals due to their contents in FA and bioactive compounds.

The publication of TALERO ELENA et al.: "Bioactive Compounds Isolated from Microalgae in Chronic Inflammation and Cancer", in the journal MARINE DRUGS, vol. 13, no. 10, 1 October 2015, pages 6152-6209, refers to microalgae including Isochrysin as source for bioactive compounds for use in inflammatory bowl diseases and colon cancer. The effect of eicosapentaenoic acid and docosahexaenoic acid, derived from microalgae, in the inflammatory process is demonstrated. Suitable microalgae are those that can be grown on a large scale e.g. Isocrysis among others. It is described that microalgae are rich in high fatty acids, proteins, antioxidant pigments and polysaccharides. Inflammasome activation and the role in carcinogenesis is shown.

The publication of NUNO K. et al.: "Effects of the marine microalgae Isochrysis galbana and Nannochloropsis oculata in diabetic rats", in the JOURNAL OF FUNCTIONAL FOODS, ELSEVIER BV, NL; INTERNATIONAL FOOD INFORMATION SERVICE, FRANKFURT-MAIN, Germany, vol. 5, no. 1, 1 January 2013, pages 106-115 reports on marine microalgae Isochrysis galbana and Nannochloropsis oculata, known to have high soluble and insoluble polysaccharide and protein contents as well as significant percentages of polyunsaturated fatty acids. Isochrysis galbana contains high amounts of docosahexaenoic acid and eicosapentaenoic acid. In diabetic subjects, these polysaccharides and fatty acids may aid in regulating glucose, lipids, lipoproteins and nitrogen compounds in endocrine metabolism, as well as increasing beneficial gastrointestinal bacteria, thus contributing to modulation of mucosa dysfunction and intestinal epithelium maintenance.

The patent application US 2020/268816 A1 describes a food supplement which is prepared from a Tisochrysis lutea extract for the treatment of non-pathological disorders, including hyperactivity, attention and memory deficit, language retardation and anxious behavior.

Accordingly, there is increasing need for an alternative capable of inhibiting inflammation and regenerating the mucous membrane of the colon. Moreover, there is a need to develop a therapeutic agent derived from a natural product having almost no side effects rather than a chemical compound that may cause severe side effects.

(Patent Document 1) Korean Patent Laid-open Publication No. 10-2018-0030274

The above information disclosed in this Background section is only for enhancement of understanding of the background of the invention, and therefore it may contain information that does not form the prior art that is already known in this country to a person of ordinary skill in the art.

### SUMMARY OF THE DISCLOSURE

The present invention is defined by the claims.

In order to solve the above problems, while searching for a composition for preventing and treating inflammatory bowel disease in natural products, the present inventors found that inflammatory bowel disease can be treated, prevented, and ameliorated based on suppression of inflammatory factors and regeneration of colonic mucosa using a natural product, when orally administering a natural product, namely the microalgae *Tisochrysis lutea,* and the present invention was completed based thereon.

Accordingly, it is one object of the present invention to provide Tisochrysis lutea as an active ingredient for use in preventing, ameliorating or treating an inflammatory bowel disease, wherein the *Tisochrysis lutea* is a lyophilized powder form, and a method of preparing the same (not part of the invention).

It is another object of the present invention to provide a health functional food comprising Tisochrysis lutea as an active ingredient for use in ameliorating or preventing an inflammatory bowel disease.

In view of the objects described above, the present invention provides the following compositions.

In one aspect, the present invention provides Tisochrysis lutea as an active ingredient for use in ameliorating, preventing or treating an inflammatory bowel disease, wherein the *Tisochrysis lutea* is a lyophilized powder form.

The active ingredient may inhibit one or more of 1L-1β, TNF-α, and NLRP3.

The active ingredient may activate one or more of ZO-1 and MUC2.

The active ingredient may inhibit one or more of 1L-1β, TNF-α, and NLRP3, and may activate one or more of ZO-1 and MUC2.

The inflammatory bowel disease may include one or more selected from the group consisting of Crohn's disease, ulcerative colitis, enteric Behcet's disease, intestinal tuberculosis enteritis, and diarrhea.

The *Tisochrysis lutea* is in the form of a lyophilized powder.

The active ingredient may be orally administered.

The active ingredient may ameliorate or treat an inflammatory bowel disease through inhibition of inflammation and regeneration of the mucous membrane of the large intestine.

In another aspect, a health functional food composition comprising Tisochrysis lutea as an active ingredient for use in ameliorating or preventing an inflammatory bowel disease is provided Additionally, In yet another aspect, a feed comprising Tisochrysis lutea as an active ingredient for use in ameliorating, preventing, or treating an inflammatory bowel disease in livestock or companion animals is provided.

The inflammatory bowel disease may include one or more selected from the group consisting of Crohn's disease, ulcerative colitis, enteric Behcet's disease, intestinal tuberculosis enteritis, and diarrhea.

Other aspects and preferred embodiments of the invention are discussed infra.

### BRIEF DESCRIPTION OF THE DRAWINGS

The above and other features of the present invention will now be described in detail with reference to certain exemplary embodiments thereof, illustrated in the accompanying drawings which are given hereinbelow by way of illustration only, and thus are not limitative of the present invention, and wherein:
FIG. 1 illustrates the result of weight measurement in Example 2-2-1 of the present invention;
FIG. 2 illustrates the result of DAI measurement in Example 2-2-1 of the present invention;
FIG. 3 illustrates the result of DAI measurement in Example 2-2-1 of the present invention;
FIG. 4 illustrates the result of measurement of the length of the large intestine in Example 2-2-2 of the present invention;
FIG. 5 illustrates the result of measurement of the length of the large intestine in Example 2-2-2 of the present invention;
FIG. 6 illustrates the result of measurement of a change in intestinal mucosal thickness in Example 2-3-1 of the present invention;
FIG. 7 illustrates the result of measurement of a change in intestinal mucosal thickness in Example 2-3-1 of the present invention;
FIG. 8 illustrates the result of verifying the change in the mucous barrier of the large intestine in Example 2-3-2 of the present invention through periodic acid-Schiff reaction (PAS) staining, the result of observing the change in the mast cells in the colon tissue in Example 2-3-3 of the present invention, and the result of ZO-1 antibody reaction in Example 2-3-4 of the present invention;
FIG. 9 illustrates the result of detecting the amelioration of inflammation in IL-1β among inflammatory cytokines through RNA analysis in Example 2-4;
FIG. 10 illustrates the result of detecting the amelioration of inflammation in TNF-α among inflammatory cytokines through RNA analysis in Example 2-4;
FIG. 11 illustrates ZO-1, the tight junction protein-related gene of Example 2-4;
FIG. 12 illustrates the result of detecting NLRP3 inhibition, meaning amelioration of inflammation through RNA analysis of Example 2-4;
FIG. 13 illustrates the result of detecting caspase-1 inhibition, meaning amelioration of inflammation through RNA analysis of Example 2-4; and
FIG. 14 illustrates the result of detecting a MUC2 gene of Example 2-4.

### DETAILED DESCRIPTION

Unless the context clearly indicates otherwise, all numbers, figures, and/or expressions that represent ingredients, reaction conditions, polymer compositions, and amounts of mixtures used in the specification are approximations that reflect various uncertainties of measurement occurring inherently in obtaining these figures, among other things. For this reason, it should be understood that, in all cases, the term "about" should be understood to modify all such numbers, figures, and/or expressions. In addition, when numerical ranges are disclosed in the description, these ranges are continuous, and include all numbers from the minimum to the maximum, including the maximum within each range, unless otherwise defined. Furthermore, when the range refers to an integer, it includes all integers from the minimum to the maximum, including the maximum within the range, unless otherwise defined.

It should be understood that, in the specification, when a range is referred to regarding a parameter, the parameter encompasses all figures including end points disclosed within the range. For example, the range of "5 to 10" includes figures of 5, 6, 7, 8, 9, and 10, as well as arbitrary sub-ranges, such as ranges of 6 to 10, 7 to 10, 6 to 9, and 7 to 9, and any figures, such as 5.5, 6.5, 7.5, 5.5 to 8.5, and 6.5 to 9, between appropriate integers that fall within the range. In addition, for example, the range of "10% to 30%" encompasses all integers that include numbers such as 10%, 11%, 12%, and 13%, as well as 30%, and any sub-ranges, such as 10% to 15%, 12% to 18%, or 20% to 30%, as well as any numbers, such as 10.5%, 15.5%, and 25.5%, between appropriate integers that fall within the range.

Hereinafter, the present invention will be described in more detail.

In the present specification, *Tisochrysis lutea* is marine microalgae that inhabits seawater and belongs to the edible algae family. It is known to be brown and about 4 to 6 µm in size.

In the present invention, an inflammatory disease is any condition specified as a local or systemic biodefense reaction caused by external physical and chemical stimuli or external infectious agents such as bacteria, fungi, viruses, and various allergens or other causes. This reaction may be caused by activation of various inflammatory mediators and immune cells, accompany a series of complicated physiological reactions such as activation of related enzymes (e.g., iNOS, COX-2, etc.), secretion of inflammatory mediators (e.g., NO, TNF-α, IL-6, IL-1β and PGE2), body fluid infiltration, cell migration, and tissue destruction, and appear externally due to symptoms such as erythema, pain, edema, fever, and deterioration or loss of specific functions of the body.

The inflammatory disease may be acute, chronic, ulcerative, allergic or necrotic. Thus, the inflammatory disease may be acute, chronic, ulcerative, allergic or necrotic, as long as the disease is considered an inflammatory disease.

In addition, in the present invention, the inflammatory disease may include an inflammatory bowel disease.

The term "inflammatory bowl disease (IBD)" refers to a chronic inflammatory disease of unknown cause that invades the digestive tract, and is an incurable disease in which diarrhea and bloody stool continue in the long term, and subsequent relapses occur. Inflammatory bowel disease may be largely divided into ulcerative colitis (UC) and Crohn's disease (CD). Although the two diseases have different clinical features, both have a chronic course, and the cause and pathophysiology are unknown, so they are collectively referred to as "inflammatory bowel disease" for convenience.

Inflammatory bowel disease is usually ulcerative colitis or Crohn's disease, but intestinal Behcet's disease, which is relatively common in Korea, also falls within the category of inflammatory bowel disease. Behcet's disease may affect multiple organs, including the skin, blood vessels, gastrointestinal tract, central nervous system, heart, and lungs, in addition to causing oral ulcers, genital ulcers, and ocular symptoms.

"Crohn's disease (CD)" and "ulcerative colitis (UC)" are chronic inflammatory bowel diseases of unknown etiology.

Unlike ulcerative colitis, Crohn's disease may affect any part of the intestine. The most prominent feature of Crohn's disease is an enlarged granular, reddish-purple edema in the intestinal wall. As inflammation occurs, the granulomas often lose their local boundaries and integrate with surrounding tissue. Diarrhea and intestinal obstruction are the main clinical behaviors thereof. Like ulcerative colitis, the course of Crohn's disease can be persistent or recurrent, mild or severe, but unlike ulcerative colitis, Crohn's disease cannot be cured by resection of the causative intestinal section. Most patients who develop Crohn's disease require surgery at some point, but repeated medical treatment is typical because subsequent relapses are common.

"Ulcerative colitis (UC)" invades the large intestine. The disease course may be continuous or recurrent, mild or severe. The earliest lesion is inflammatory infiltration in which an abscess is formed at the base of the crypts of Lieberkühn. Adhesions of these swollen and ruptured crypts tend to separate the overlying mucosa from the blood supply thereof, causing ulceration. Symptoms of this disease include cramps, lower abdominal pain, rectal bleeding, and frequent and soft feces mainly composed of blood, pus, and mucous, along with thin stool particles. Total colectomy may be required for acute, severe, chronic, or persistent ulcerative colitis. The clinical behavior of UC is highly variable, the onset thereof may be insidious or sudden, and symptoms thereof may include diarrhea, tenesmus and recurrent rectal bleeding. With fulminant outbreaks of the entire colon, a life-threatening emergency, toxic megacolon, may occur. Symptoms that may manifest outside the intestine include arthritis, pyoderma gangrene, uveitis and erythema nodosum.

Therefore, inflammatory bowel disease, which can be treated, prevented, or ameliorated by the composition containing *Tisochrysis lutea* includes, for example, one or more selected from the group consisting of Crohn's disease, ulcerative colitis, enteric Behcet's disease, intestinal tuberculosis enteritis, and diarrhea.

In an embodiment, the active ingredient inhibits one or more of 1L-113, TNF-α, and NLRP3.

In an embodiment, the active ingredient may activate one or more of ZO-1 and MUC2.

In an embodiment, the active ingredient may inhibit one or more of 1L-1β, TNF-α, and NLRP3, and may activate one or more of ZO-1 and MUC2.

In an embodiment, the inflammatory bowel disease may include one or more selected from the group consisting of Crohn's disease, ulcerative colitis, enteric Behcet's disease, intestinal tuberculosis enteritis, and diarrhea.

The *Tisochrysis lutea* is in the form of a lyophilized powder.

In an embodiment, the active ingredient may be administered orally.

In an embodiment, the active ingredient may ameliorate or treat inflammatory bowel disease through inhibition of inflammation and regeneration of the mucous membrane of the large intestine.

The active ingredient is *Tisochrysis lutea,* and the *Tisochrysis lutea* is in the form of a lyophilized powder.

Carriers, excipients and diluents that may be contained in the pharmaceutical composition of the present invention include one or more selected from the group consisting of lactose, dextrose, sucrose, sorbitol, mannitol, xylitol, erythritol, maltitol, starch, acacia gum, alginate, gelatin, calcium phosphate, calcium silicate, cellulose, methyl cellulose, hydroxymethyl cellulose, microcrystalline cellulose, silicified microcrystalline cellulose, povidone, crospovidone, croscarmellose sodium, polyvinylpyrrolidone, water, methylhydroxybenzoate, propylhydroxybenzoate, Noisirin, colloidal silicon dioxide, lactose, talc, magnesium stearate, colloidal magnesium stearyl, and mineral oil.

The amount of the pharmaceutical composition that is administered is determined by those skilled in the art in consideration of other well-known factors in the medical field, such as the purpose of use, the severity of disease, age, weight, health condition, gender, sensitivity to drugs, administration time, administration route, or the type of material used as an active ingredient.

The subject may be a mammal. The mammal may be a human, dog, cat, cow, goat, or pig. In addition, the subject may be a human or non-human animal, and may be a human or non-human mammal. The administration may be performed as desired through any general route, as long as the composition can reach the target tissue, for example, through application to the skin, intraperitoneal administration, intravenous administration, intramuscular administration, subcutaneous administration, intradermal administration, transdermal patch application, oral administration, intranasal administration, intrapulmonary administration, rectal administration, topical administration, eye drop administration, and the like, and specifically, through eye drop administration or the like. The preferred dosage of the pharmaceutical composition varies depending on the patient's age and weight, disease severity, drug form, and administration route and period, but may be appropriately selected by those skilled in the art. However, for desirable effects, the pharmaceutical composition may be administered at 0.001 mg/kg to 1 mg/kg per day, preferably 0.1 mg/kg to 10 mg/kg per day. The administration may be performed several times a day, preferably divided into 1 to 6 doses, at regular time intervals according to the prescription of a doctor or pharmacist.

In another aspect, the present invention provides a feed composition for use in treating, preventing or ameliorating an inflammatory bowel disease in livestock or companion animals containing *Tisochrysis lutea* as an active ingredient.

In another aspect, the present invention provides a health functional food composition for use in ameliorating or preventing an inflammatory bowel disease containing *Tisochrysis lutea* as an active ingredient.

In an embodiment, the inflammatory bowel disease includes at least one selected from the group consisting of Crohn's disease, ulcerative colitis, enteric Behcet's disease, enteric tuberculosis enteritis, and diarrhea.

The health food composition of the present invention contains *Tisochrysis lutea,* and there is no particular limitation as to the type thereof. Examples of the food include drinks, meat, sausage, bread, biscuits, rice cakes, Sunsik (Korean ready-to-eat food prepared from grains), chocolate, candy, snacks, confectioneries, pizza, ramen, other noodles, gums, dairy products including ice cream, various soups, beverages, alcoholic beverages, vitamin complexes, dairy products, and processed dairy products, and include all other functional health foods in the conventional sense. As an active ingredient, a *Tisochrysis lutea* powder or dried powder thereof may be added alone to the food, or may be used in conjunction with other foods or food ingredients, and may be suitably used according to conventional methods. The effective content may be appropriately determined according to the purpose of use (for prevention or amelioration), and may be present in a range of 0.001 to 70% by weight with respect to the total weight of the health food. However, in the case of long-term intake for health and hygiene purposes or for health control, the amount may be below the above range, and the active ingredient may be also used in an amount above the range, since there is no problem in terms of safety. For example, in the case of preparing health beverages, the health drink may contain, in addition to the active ingredient, natural carbohydrates or flavoring agents, which are additives commonly used in the preparation of beverages. The natural carbohydrates may include conventional sugars, such as monosaccharides (e.g. glucose, fructose, etc.), disaccharides (e.g. maltose, sucrose, etc.) and polysaccharides (e.g., dextrin, cyclodextrin, etc.), and sugar alcohols such as xylitol, sorbitol and erythritol. The natural carbohydrate may be present in a range of 1 to 20% by weight, preferably 5 to 10% by weight, with respect to the total weight of the health food. The flavoring agent may include natural flavoring agents (thaumatin, stevia extract, rebaudioside A, glycyrrhizin, etc.) and synthetic flavoring agents (saccharin, aspartame, etc.). The health food may contain other nutrients, vitamins, minerals (electrolytes), flavors (synthetic or natural flavors), colorants, pectic acids and salts thereof, alginic acids and salts thereof, organic acids, protective colloidal thickeners, pH-adjusting agents, stabilizers, preservatives, glycerin, alcohol, carbonic acid used in carbonated beverages, and the like. In addition, it may contain flesh for the production of natural fruit juices, fruit juice beverages, and vegetable beverages. The content of these additives is not particularly limited, but may fall within a range of 0.1 to 20% by weight with respect to the total weight of the health food.

The active ingredient contained in the composition according to one or another aspect of the present invention may be present in an amount of 0.01% to 50% by weight based on the total weight of the composition.

Hereinafter, the present invention will be described in more detail with reference to specific examples. However, the following examples are provided only for better understanding of the present invention, and thus should not be construed as limiting the scope of the present invention which is only limited by the scope of the claims.

### Example 1. Preparation of Isochrysis aff. galbana

*Isochrysis aff. galbana (I. aff. galbana)* was obtained through IsoPrime manufactured by Proviron Corp. in Belgium.

The *Isochrysis aff. galbana* was in the form of a lyophilized brown fine powder.

### Example 2. Study on efficacy of Isochrysis aff. galbana for inflammatory bowel disease

The efficacy of I. *aff. galbana* in ameliorating an inflammatory bowel disease in animal models (C57BL/6 male mice) was investigated. The efficacy of ameliorating an inflammatory bowel disease was investigated using a total of 15 animals (n = 5 in each experimental group).

### Example 2-1. Conditions for treatment of animal models to evaluate efficacy of ameliorating inflammatory bowel disease

The experimental groups included a total of 3 groups, namely, an untreated group (control), an inflammatory bowel disease-induced group (DSS induced model), and a group administered with 300 mg/kg/day of I. *aff. galbana* after induction of inflammatory bowel disease (DSS induced model with I. *aff. galbana).*

The experiment was performed as follows. Enteritis was induced for 5 days by providing unlimited drinking water with an aqueous solution containing 2% (wt/v) dextran sulfate (DSS) for 7 days to induce inflammatory bowel disease. After induction of inflammatory bowel disease, *I. aff. Galbana* was orally administered in an amount of 300 mg/kg/day for 5 days, during which time the untreated group and the intestinal disease-induced group were orally administered with drinking water.

### Example 2-2. Evaluation of enteritis in DSS-induced inflammatory bowel disease animal model

### Example 2-2-1. Evaluation of disease activity

In order to clinically evaluate DSS-induced enteritis, the extent of weight loss, stool hardness, and presence of gloss bloody stool were observed daily, and scores from 0 to 4 points were assigned depending on the state of these items during the experiment. The disease activity index (DAI) was assessed as:
0, no weight loss, normal loose stool, no bloody stool;
2, 5-10% weight loss, slightly loose stool, brown stool;
3, 10-20% weight loss, loose stool, red stool;
4, at least 20% weight loss, diarrhea, bloody stool.

Disease activity was calculated by adding up the scores for respective weight loss, stool hardness, and gross bloody stool items and then dividing the result by 3.

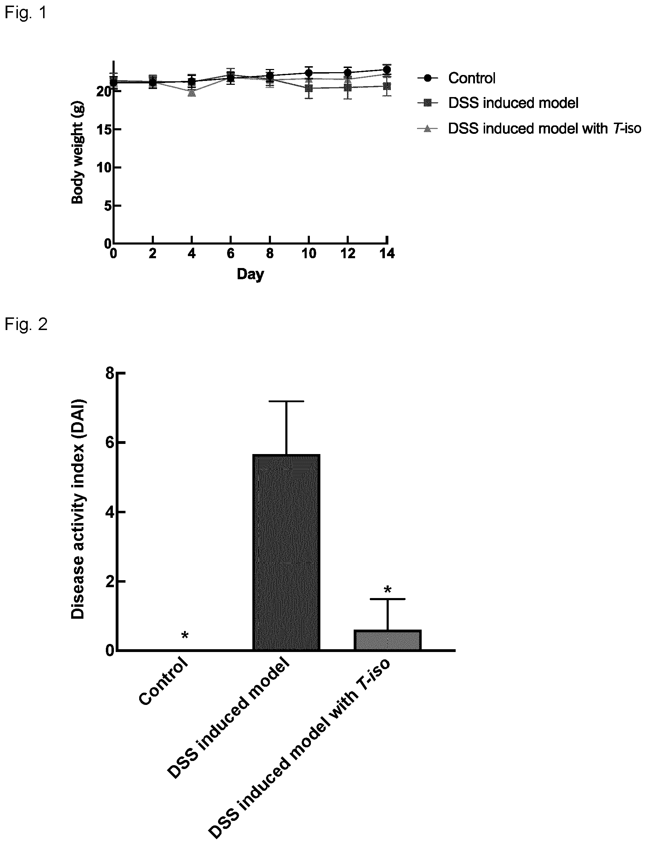

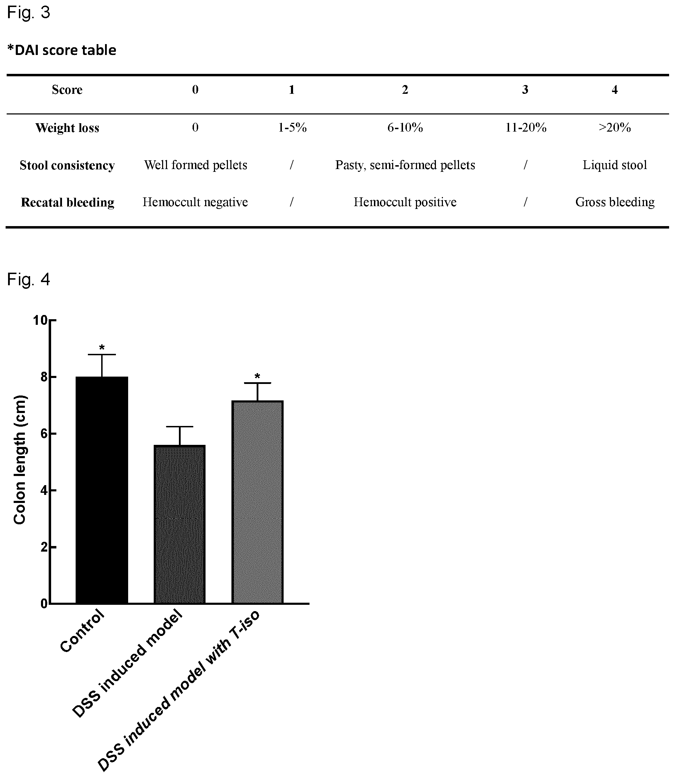

The results were as shown in FIGS. 1 to 3. FIG. 1 illustrates the result of weight measurement, and FIGS. 2 and 3 illustrate the results of DAI measurement.

### Example 2-2-2. Measurement of length of large intestine

In order to determine the effects on DSS-induced enteritis, the length of the changed colon was measured. The measurement was performed by administering I. *aff. galbana* to mice, sacrificing the mice, extracting the large intestine after laparotomy, and measuring the length from the cecum to the anus using a Vernier caliper.

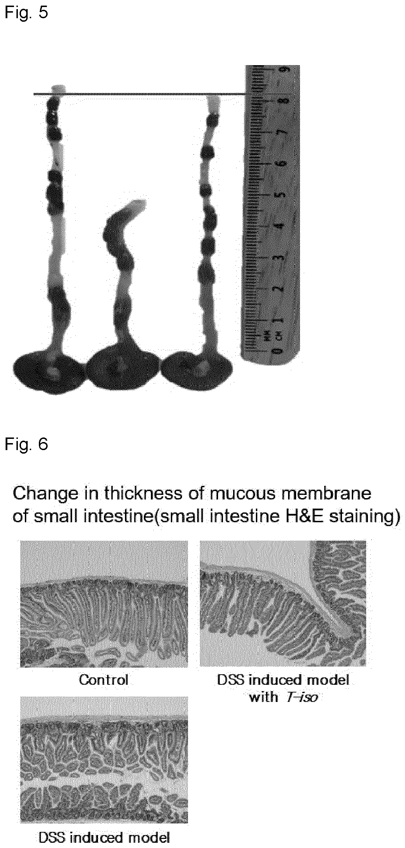

The obtained image and results were shown in FIGS. 4 and 5. As can be seen from FIGS. 4 and 5, the length of the shortened intestine is restored after administration of I. *aff. galbana.*

### Example 2-3. Evaluation of efficacy of ameliorating inflammatory bowel disease through histological analysis

For histological analysis, the large intestine was extracted, the length from the cecum to the anus was measured, the contents of the large intestine were washed with physiological saline, and water was removed therefrom. Small and large intestine tissues were partially fixed in 10% formalin. The fixed tissue was embedded in paraffin using a conventional method, and was then prepared into a continuous section having a thickness of 5 µm.

### Example 2-3-1. Verification of efficacy in ameliorating inflammatory bowel disease through changes in thickness of intestinal mucosa

The small intestine tissues prepared in sections were stained with hematoxylin & eosin and then observed at a magnification of 40x using an optical microscope. Mucosal thickness was expressed as the average of values measured at several sites.

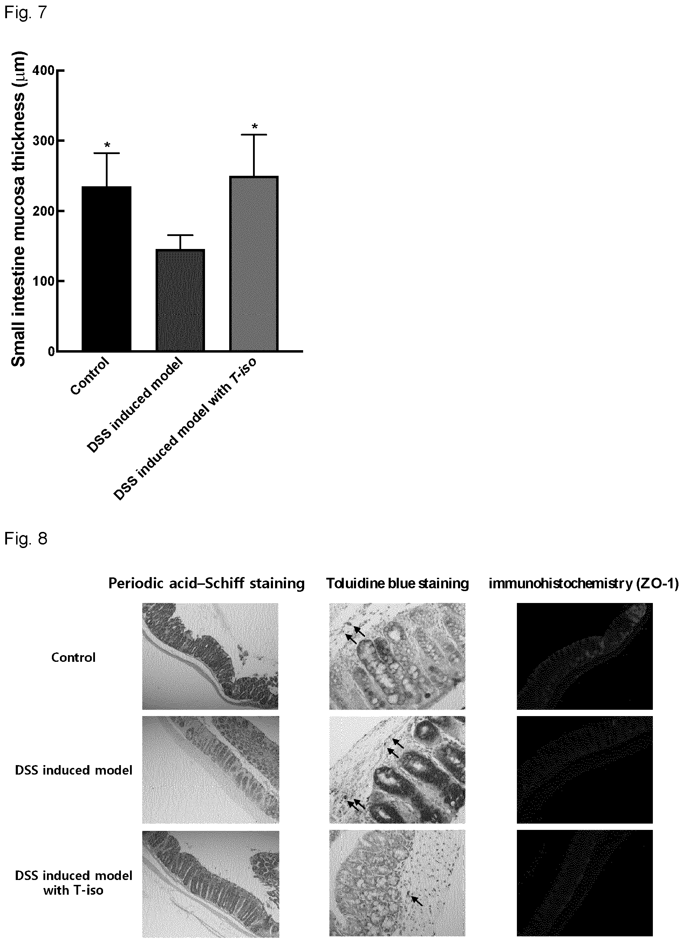

The change in the thickness of the intestinal mucosa is shown in FIGS. 6 and 7.

### Example 2-3-2. Verification of efficacy in ameliorating inflammatory bowel disease through changes in mucous barrier of large intestine (periodic acid-Schiff reaction staining, PAS)

Periodic acid-Schiff reaction (PAS) staining was performed to examine changes in mucin-secreting cells that secrete neutral mucin forming a mucous barrier. First, the cells were treated with periodic acid for 5 minutes and then treated with Schiff reagent for 15 minutes. Then, the cells were washed 3 times with a sulfurous rinse solution for 2 minutes each time, and counterstained in hematoxylin for 1 minute.

The experimental results were shown in FIG. 8.

### Example 2-3-3. Verification of efficacy of ameliorating inflammatory bowel disease by observation of change in mast cells in colon tissue (toluidine blue staining)

Paraffin-fixed tissue sections were deparaffinized with xylene, hydrated in a series of decreasing concentrations of alcohol, and washed with distilled water. The sections were reacted with 0.05% toluidine blue dissolved in citrate-phosphate buffer (pH 4.0)) at room temperature for 10 minutes, washed with distilled water, dehydrated and subjected to a transparent process, and observed through an optical microscope.

The experimental results were shown in FIG. 8.

### Example 2-3-4. Verification of efficacy of ameliorating inflammatory bowel disease through immunofluorescence staining (immunohistochemistry ZO-1)

The colon tissue sections were washed with PBS buffer for 10 minutes and antigen retrieval was performed in a microwave oven using 10 mM Tris /1 mM EDTA buffer (pH 9.0) 3 times for 2 minutes each, at intervals of 5 minutes. After the antigen retrieval, the result was allowed to stand in a 1% BSA (in PBS) solution at room temperature for 1 hour after being cooled to room temperature in order to reduce nonspecific reactions. The ZO-1 antibody was used as a primary antibody. After reaction with the antibody for half a day, the reaction was performed at room temperature using a DAKO EnVision kit for 1 hour, followed by reaction using a diaminobenzidine substrate kit for 1 to 2 minutes. After treatment with VECTASHIELD medium containing DAPI, the result was covered with a cover slide and observed using a confocal microscope.

The experimental results were shown in FIG. 8.

### Example 2-4. Evaluation of effect of ameliorating inflammation at RNA level through animal experiment

200 mg of the extracted tissue was assayed and homogenized using 400 µL of buffer (100 mM Tri-HCl, 250 mM sucrose pH 7.4) added therewith. After homogenization, the result was placed in a new tube and centrifuged at 10,000 xg for 10 minutes. The middle layer, excluding the debris of the upper and lower layers, was isolated and used as a sample. The content of each of IL-1β, TNF-α, ZO-1, NLRP3, Caspase-1, and MUC2 in the prepared samples was measured by enzyme-linked immunosorbent assay (ELISA). Absorbance at 450 nm was measured with an ELISA reader, and then the cytokine concentration was quantified using a standard curve.

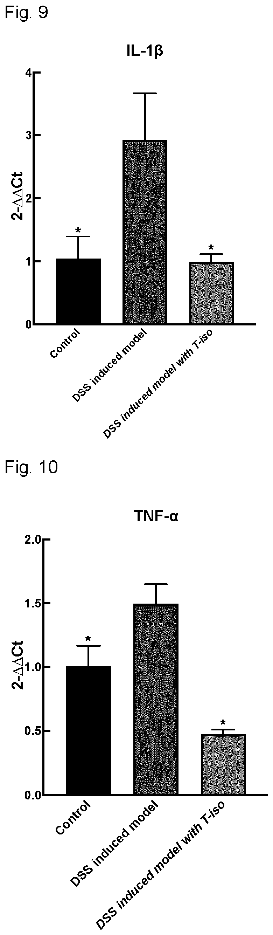

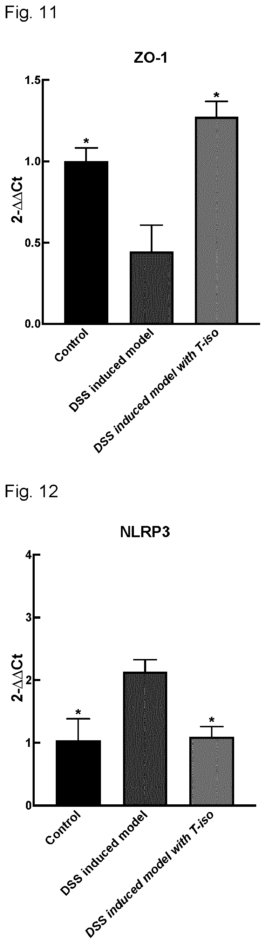

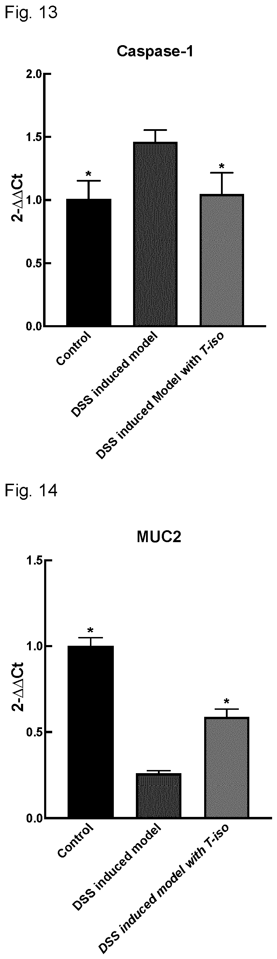

The experimental results are shown in FIGS. 9 to 14.

Hereinafter, formulation examples of the composition according to the present invention will be described. However, these formulation examples are provided only for better understanding of the present invention, and thus should not be construed as limiting the scope of the present invention.

### [Formulation Example 1] Preparation of pill

30% by weight of Example 1, 30% by weight of corn starch, 20% by weight of glycerin, and 20% by weight of a sorbitol powder were mixed, and a pill was produced using a pill-making machine. The final weight of the product was 3.5 g.

### [Formulation Example 2] Preparation of tablet

30% by weight of the extract of Example 1, 20.5% by weight of lactose, 20% by weight of dextrin, 20% by weight of a maltitol powder and 7% by weight of a xylitol powder were mixed, granulated using a fluidized bed dryer, added with 2.5% sugar ester by weight, and then tableted with a tableting machine. The final weight of the product was 2 g.

### [Formulation Example 3] Preparation of granules

30% by weight of the extract of Example 1, 5% by weight of xylitol, and 65% by weight of isomalt were mixed, formed into granules using a fluidized bed granulator, and then placed in a bag. The final weight of the product was 2 g.

### [Formulation Example 4] Health food

A health food having the composition shown in Table 1 below was prepared in a conventional manner.

**[Table 1]**

| Ingredient to be mixed | Content |
|---|---|
| Example 1 | 20 mg |
| Vitamin A acetate | 70 µg |
| Vitamin E | 1.0 mg |
| Vitamin B1 | 0.13 mg |
| Vitamin B2 | 0.15 mg |
| Vitamin B6 | 0.5 mg |
| Vitamin B12 | 0.2 µg |
| Vitamin C | 10 mg |
| Biotin | 10 µg |
| Nicotinic acid amide | 1.7 mg |
| Folic acid | 50 µg |
| Calcium pantothenate | 0.5 mg |
| Ferrous sulfate | 1.75 mg |
| Zinc oxide | 0.82 mg |
| Magnesium carbonate | 25.3 mg |
| Monobasic potassium phosphate | 15 mg |
| Dicalcium phosphate | 55 mg |
| Potassium citrate | 90 mg |
| Calcium carbonate | 100 mg |
| Magnesium chloride | 24.8 mg |

### [Formulation Example 5] Health beverage

A health beverage having the composition shown in Table 2 below was prepared in a conventional manner.

**[Table 2]**

| Ingredient to be mixed | Content |
|---|---|
| Example 1 | 1000 mg |
| Citric acid | 1000 mg |
| Oligosaccharide | 100 g |
| Taurine | 1 g |
| Purified water | Balance |

The present invention is only limited by the scope of the claims.

It should be understood that the effects of the present invention include all effects that can be inferred from the description of the present invention and falling under the scope of the claims.

The present invention has been described in detail with reference to embodiments thereof.

## Claims

1. A *Tisochrysis lutea* as an active ingredient for use in ameliorating, preventing, or treating an inflammatory bowel disease, wherein the *Tisochrysis lutea* is a lyophilized powder form.

2. The *Tisochrysis lutea* according to the use of claim 1, wherein the active ingredient inhibits one or more of 1L-1β, TNF-α and NLRP3.

3. The *Tisochrysis lutea* according to the use of claim 1 or 2, wherein the active ingredient activates one or more of ZO-1 and MUC2.

4. The *Tisochrysis lutea* according to the use of one of claims 1 to 3, wherein the active ingredient inhibits one or more of 1L-113, TNF-α and NLRP3, and activates one or more of ZO-1 and MUC2.

5. The *Tisochrysis lutea* according to the use of one of claims 1 to 4, wherein the inflammatory bowel disease comprises one or more selected from the group consisting of Crohn's disease, ulcerative colitis, enteric Behcet's disease, intestinal tuberculosis enteritis, and diarrhea.

6. The *Tisochrysis lutea* according to the use of one of claims 1 to 5, wherein the active ingredient is administered orally.

7. The *Tisochrysis lutea* according to the use of one of claims 1 to 6, wherein the active ingredient ameliorates or treats the inflammatory bowel disease through inhibition of inflammation and regeneration of the mucous membrane of the large intestine.

8. A health functional food comprising *Tisochrysis lutea* as an active ingredient for use in ameliorating or preventing an inflammatory bowel disease.

9. The health functional food according to claim 8, wherein the inflammatory bowel disease comprises one or more selected from the group consisting of Crohn's disease, ulcerative colitis, enteric Behcet's disease, intestinal tuberculosis enteritis, and diarrhea.

10. A feed comprising *Tisochrysis lutea* as an active ingredient for use in ameliorating, preventing, or treating an inflammatory bowel disease in livestock or companion animals.

## Patentansprüche

1. *Tisochrysis lutea* als aktiver Bestandteil zur Verwendung bei der Linderung, Vorbeugung oder Behandlung einer entzündlichen Darmerkrankung, wobei *Tisochrysis lutea* in Form eines lyophilisierten Pulvers vorliegt.

2. *Tisochrysis lutea* gemäß der Verwendung nach Anspruch 1, wobei der aktive Bestandteil 1L-1β, TNF-α und/oder NLRP3 hemmt.

3. *Tisochrysis lutea* gemäß der Verwendung nach Anspruch 1 oder 2, wobei der aktive Bestandteil ZO-1 und/oder MUC2 aktiviert.

4. *Tisochrysis lutea* gemäß der Verwendung nach einem der Ansprüche 1 bis 3, wobei der aktive Bestandteil 1L-1β, TNF-α und/oder NLRP3 hemmt und ZO-1 und/oder MUC2 aktiviert.

5. *Tisochrysis lutea* gemäß der Verwendung nach einem der Ansprüche 1 bis 4, wobei die entzündliche Darmerkrankung mindestens eine aus der Gruppe bestehend aus Morbus Crohn, Colitis ulcerosa, Morbus Beh 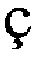 et des Darms, Darmtuberkulose und Diarrhoe ausgewählte Darmerkrankung umfasst.

6. *Tisochrysis lutea* gemäß der Verwendung nach einem der Ansprüche 1 bis 5, wobei die Verabreichung des aktiven Bestandteils oral erfolgt.

7. *Tisochrysis lutea* gemäß der Verwendung nach einem der Ansprüche 1 bis 6, wobei die entzündliche Darmerkrankung mittels des aktiven Bestandteils durch Entzündungshemmung und Regeneration der Schleimhaut des Dickdarms linderbar oder behandelbar ist.

8. Gesundheitsförderndes funktionelles Lebensmittel, umfassend *Tisochrysis lutea* als einen aktiven Bestandteil zur Verwendung bei der Linderung oder Vorbeugung einer entzündlichen Darmerkrankung.

9. Gesundheitsförderndes funktionelles Lebensmittel nach Anspruch 8, wobei die entzündliche Darmerkrankung mindestens eine aus der Gruppe bestehend aus Morbus Crohn, Colitis ulcerosa, Morbus Beh 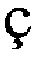 et des Darms, Darmtuberkulose und Diarrhoe ausgewählte Darmerkrankung umfasst.

10. Futtermittel, umfassend *Tisochrysis lutea* als einen aktiven Bestandteil zur Verwendung bei der Linderung, Vorbeugung oder Behandlung einer entzündlichen Darmerkrankung bei Nutztieren oder Haustieren.

## Revendications

1. *Tisochrysis lutea* comme ingrédient actif pour l'utilisation dans l'amélioration, la prévention ou le traitement d'une maladie inflammatoire de l'intestin, dans laquelle *Tisochrysis lutea* est en forme d'une poudre lyophilisée.

2. *Tisochrysis lutea* selon l'utilisation de la revendication 1, dans laquelle l'ingrédient actif inhibe un ou plusieurs de 1L-1β, TNF-α et NLRP3.

3. *Tisochrysis lutea* selon l'utilisation de la revendication 1 ou 2, dans laquelle l'ingrédient actif active un ou plusieurs de ZO-1 et MUC2.

4. *Tisochrysis lutea* selon l'utilisation de l'une quelconque des revendications 1 à 3, dans laquelle l'ingrédient actif inhibe un ou plusieurs de 1L-1β, TNF-α et NLRP3 et active un ou plusieurs de ZO-1 et MUC2.

5. *Tisochrysis lutea* selon l'utilisation de l'une quelconque des revendications 1 à 4, dans laquelle la maladie inflammatoire de l'intestin comprend une ou plusieurs maladies sélectionnées dans le groupe consistant en maladie de Crohn, rectocolite hémorragique, maladie de Behçet entérique, tuberculose intestinale et diarrhée.

6. *Tisochrysis lutea* selon l'utilisation de l'une quelconque des revendications 1 à 5, dans laquelle l'ingrédient actif est administré oralement.

7. *Tisochrysis lutea* selon l'utilisation de l'une quelconque des revendications 1 à 6, dans laquelle l'ingrédient actif améliore ou traite la maladie inflammatoire de l'intestin par inhibition d'inflammation et par régénération de la muqueuse du gros intestin.

8. Alimentation fonctionnelle saine comprenant *Tisochrysis lutea* comme ingrédient actif pour l'utilisation dans l'amélioration ou la prévention d'une maladie inflammatoire de l'intestin.

9. Alimentation fonctionnelle saine selon la revendication 8, dans laquelle la maladie inflammatoire de l'intestin comprend une ou plusieurs maladies sélectionnées dans le groupe consistant en maladie de Crohn, rectocolite hémorragique, maladie de Behçet entérique, tuberculose intestinale et diarrhée.

10. Nourriture comprenant *Tisochrysis lutea* comme ingrédient actif pour l'utilisation dans l'amélioration, la prévention ou le traitement d'une maladie inflammatoire de l'intestin dans des animaux productifs ou des animaux de compagnie.
